# EUROPÄISCHE PATENTSCHRIFT

(11) **EP 2 210 097 B1**
(45) Veröffentlichungstag und Bekanntmachung des Hinweises auf die Patenterteilung: **24.05.2017**
(21) Anmeldenummer: 08844542.4
(22) Anmeldetag: 29.10.2008
(51) Int. Cl.: G01N 33/50, G01N 33/569

(54) **VERFAHREN ZUR IN VITRO-DIAGNOSE UND/ODER IN VITRO-THERAPIEVERFOLGUNG VON INFEKTIONEN**
METHOD FOR THE IN VITRO DIAGNOSIS AND/OR IN-VITRO THERAPY MONITORING OF INFECTIONS
PROCÉDÉ DE DIAGNOSTIC IN VITRO ET/OU DE SUIVI DE TRAITEMENT IN VITRO RELATIVEMENT À DES INFECTIONS

(30) Priorität: 29.10.2007 DE 102007052518
(43) Veröffentlichungstag der Anmeldung: 28.07.2010
(73) Patentinhaber: Autoimmun Diagnostika GmbH, 72479 Strassberg (DE)
(72) Erfinder: SCHÖLLHORN, Volkmar, 72458 Albstadt (DE)
(74) Vertreter: Patentanwälte Ruff, Wilhelm, Beier, Dauster & Partner mbB
(86) Internationale Anmeldenummer: PCT/EP2008/009113
(87) Internationale Veröffentlichungsnummer: WO 2009/056283

(56) Entgegenhaltungen:
- US-A1- 2005 089 942
- PEI J ET AL: "Specific antibody secreting cells from chickens can be detected by three days and memory B cells by three weeks post-infection with the avian respiratory coronavirus" DEVELOPMENTAL AND COMPARATIVE IMMUNOLOGY, PERGAMON PRESS, US, Bd. 29, Nr. 2, 1. Januar 2005 (2005-01-01), Seiten 153-160, XP004574974 ISSN: 0145-305X
- BOECHER W O ET AL: "Regulation of the neutralizing anti-hepatitis B surface (HBs) antibody response in vitro in HBs vaccine recipients and patients with acute or chronic hepatitis B virus (HBV) infection" CLINICAL AND EXPERIMENTAL IMMUNOLOGY, Bd. 105, Nr. 1, 1996, Seiten 52-58, XP002509674 ISSN: 0009-9104
- NILSSEN D E ET AL: "B-cell activation in duodenal mucosa after oral cholera vaccination in IgA deficient subjects with or without IgG subclass deficiency" SCANDINAVIAN JOURNAL OF IMMUNOLOGY, Bd. 38, Nr. 2, 1993, Seiten 201-208, XP002510247 ISSN: 0300-9475
- CZERKINSKY C C ET AL: "A solid-phase enzyme-linked immunospot (ELISPOT) assay for enumeration of specific antibody-secreting cells" JOURNAL OF IMMUNOLOGICAL METHODS, ELSEVIER SCIENCE PUBLISHERS B.V.,AMSTERDAM, NL, Bd. 65, Nr. 1-2, 16. Dezember 1983 (1983-12-16), Seiten 109-121, XP023973707 ISSN: 0022-1759 [gefunden am 1983-12-16]

## Beschreibung

Die vorliegende Erfindung betrifft ein Verfahren zur in vitro-Diagnose und/oder in vitro-Therapieverfolgung von Infektionen und/oder Infektionserkrankungen, insbesondere zur Unterscheidung zwischen akuten Infektionen einerseits und latenten oder überstandenen Infektionen andererseits, sowie ein entsprechendes Kit.

Zur Erkennung körpereigener Strukturen und zur Abwehr körperfremder Organismen oder Stoffe verfügen höhere Organismen, wie Tiere oder Menschen, über ein hochkomplexes Immunsystem. Das menschliche Immunsystem lässt sich in zwei Gruppen von Abwehrmechanismen unterteilen. Es wird zwischen einer humoralen sowie einer zellulären Immunabwehr unterschieden. Eine angeborene Immunabwehr wird als natürliche Immunität bezeichnet. Bildet sich die Immunität erst nach Kontakt mit als körperfremd erkannten Strukturen aus, sogenannten Antigenen, wird sie als erworbene Immunität bezeichnet. Für solche streng spezifischen Mechanismen spielen Leukozyten, wie Monozyten, Granulozyten sowie T- und B-Lymphozyten, sowohl in der zellulären als auch in der humoralen Abwehr eine wichtige Rolle.

Im Falle von Infektionen oder Infektionserkrankungen ist der Organismus in der Lage auf eine Vielzahl von Antigenen zu reagieren. Durch eine Proliferation der B-Lymphozyten, insbesondere eine klonale Expansion, in Kombination mit einer Selektion von weiteren Abwehrzellen unter Einbeziehung von Differenzierungsvorgängen kann der Organismus in der Regel den eingedrungenen Antigenen mit neutralisierenden Antikörpern begegnen.

In der Praxis stellt sich einem Mediziner für eine Diagnose jedoch häufig das Problem, zwischen akuten, latenten oder zurückliegenden Infektionen oder damit zusammenhängenden Infektionserkrankungen zu unterscheiden. Eine Unterscheidung zwischen gerade eingetretenen (akuten bzw. aktiven) Infektionen und latenten bzw. chronischen oder bereits zurückliegenden Infektionen ist beispielsweise für eine Einschätzung entscheidend, ob infizierte oder erkrankte Patienten noch infektiös sind.

Zur Unterscheidung der verschiedenen Phasen von Infektionen oder damit im Zusammenhang stehenden Folgeerkrankungen bietet sich grundsätzlich ein direkter Nachweis von Infektionserregern oder Teilen davon an. Allerdings besteht hierbei zum einen die Schwierigkeit, die Infektionserreger zu isolieren. Zum anderen müssen die isolierten Infektionserreger mit Hilfe geeigneter Kulturverfahren gezüchtet werden, damit ein einwandfreier Nachweis möglich ist. Dies ist gewöhnlich mit einem hohen Kosten- und Zeitaufwand verbunden und zudem nicht für alle Erreger durchführbar.

Für eine indirekte Form des Infektionsnachweises kommen grundsätzlich auch Antikörper in Betracht, die gegen die Infektionserreger gebildet werden. Nachteilig ist hierbei allerdings, dass sich Antikörper gewöhnlich auch noch Jahre nach einer überstandenen Infektion im Blut von Patienten nachweisen lassen. Deswegen ist auch in diesem Fall insbesondere keine Unterscheidung zwischen einer akuten, latenten oder überstandenen Infektion möglich. Für sich gegebenenfalls anschließende Therapiemaßnahmen wäre es jedoch wünschenswert, wenn in diagnostischer Hinsicht eine Aussage über den Infektionsstatus (akut, latent oder überstanden) getroffen werden könnte.

Nachweisverfahren für den für respiratorische Infektionen verantwortlichen Coronavirus bei Hühnern sind bekannt (Pei J. et al.: Specific antibody secreting cells from chickens can be detected by three days and memory B cells by three weeks post-infection with the avian respiratory coronavirus", Developmental & Comparative Immunology 29, 2005: 153-160).

Aus der US 2005/0089942 A1 sind Nachweisverfahren für Infektionserkrankungen, insbesondere auch für Tuberkulose, bekannt.

Weiterhin ist der Nachweis von B-Zellen, welche gegen das Hepatitis-B-Virus gerichtete Antikörper produzieren, bekannt (Böcher et al.: "Regulation of the neutralizing anti-hepatitis B surface (HBs) antibody response in vitro in HBs vaccine recipients and patients with acute or chronic hepatitis B virus (HBV) infection, Clin Exp Immunol. 1996, 105: 52-58).

Weiterhin ist die Aktivierung von B-Zellen in der Schleimhaut des Zwölffingerdarms nach einer oralen Cholera-Impfung bekannt (Nilssen et al.: "B-cell Activation in Duodenal Mucosa after Oral Cholera Vaccination in IgA Deficient Subjects with or without IgG Subclass Deficiency", Scand. J. Immunol. 38, 1993: 201-208).

Ferner ist der Nachweis von spezifische Antikörper sezernierenden Zellen auf Basis eines ELISPOT-Assays bekannt (Czerkinsky et al.: "A Solid-Phase Enzyme-Linked Immunospot (ELISPOT) Assay for Enumeration of Specific Antibody-Secreting Cells, Journal of Immunological Methods, 65, 1983: 109-121).

Die vorliegende Erfindung stellt sich daher die Aufgabe, ein zuverlässiges Verfahren zur in vitro-Diagnose und/oder in vitro-Therapieverfolgung bereitzustellen, welches vor allem eine Unterscheidung im Hinblick auf den Infektionsstatus erlaubt.

Diese Aufgabe wird erfindungsgemäß gelöst durch ein Verfahren mit den Merkmalen des unabhängigen Anspruchs 1. Bevorzugte Ausführungsformen des Verfahrens sind Gegenstand der abhängigen Ansprüche 2 bis 9. Weiterhin betrifft die vorliegende Erfindung auch Verwendungen des Verfahrens gemäß den Ansprüchen 10 und 11. Ein weiterer Aspekt der Erfindung betrifft die Verwendung eines Kits mit den Merkmalen des Anspruchs 12.

Bei dem erfindungsgemäßen Verfahren handelt es sich um ein Verfahren zur in vitro-Diagnose und/oder in vitro-Therapieverfolgung von Infektionen und/oder Infektionserkrankungen zur Unterscheidung zwischen akuten Infektionen bzw. Infektionserkrankungen einerseits und latenten oder überstandenen Infektionen bzw. Infektionserkrankungen andererseits, wobei eukaryotische Zellen, vorzugsweise auf einer Untersuchungsfläche bzw. auf einem Träger, mit einem Antigen inkubiert werden und auf Antigen spezifische Antikörper sezernierende Zellen (ASCs) getestet werden, deren sezernierte Antikörper spezifisch gegen das Antigen gerichtet sind.

Mit anderen Worten handelt es sich bei dem erfindungsgemäßen Verfahren um ein in vitro-Verfahren zur Diagnose und/oder Therapieverfolgung von Infektionen und/oder Infektionserkrankungen zur Unterscheidung zwischen akuten Infektionen bzw. Infektionserkrankungen einerseits und latenten oder überstandenen Infektionen bzw. Infektionserkrankungen andererseits, wobei eukaryotische Zellen, vorzugsweise auf einer Untersuchungsfläche bzw. auf einem Träger, mit einem Antigen inkubiert werden und auf Antigen spezifische Anitkörper sezernierende Zellen (ASCs) getestet werden, deren sezernierte Antikörper spezifisch gegen das Antigen gerichtet sind.

Durch die Erfindung wird ein in vitro-Verfahren bereitgestellt, welches eine Unterscheidung zwischen akuten (aktiven) Infektionen einerseits und latenten (chronischen) oder überstandenen Infektionen andererseits erlaubt. Im Rahmen des erfindungsgemäßen Verfahrens werden hierzu eukaryotische Zellen auf die Anwesenheit von ASCs untersucht, deren ausgeschiedene Antikörper gegen das Antigen gerichtet sind, das zur Inkubierung der eukaryotischen Zellen verwendet wird. Bei den mit Hilfe des erfindungsgemäßen Verfahrens nachweisbaren ASCs handelt es sich in der Regel um Effektor-Zellen, insbesondere Effektor-B-Zellen. Dieser besondere Zelltyp erlaubt eine Differenzierung zwischen akuten (aktiven) Infektionen einerseits und latenten (chronischen) oder überstandenen Infektionen andererseits. Werden die eukaryotischen Zellen positiv auf ASCs getestet, so stammen die eukaryotischen Zellen von einem menschlichen oder tierischen Organismus, der an einer akuten Infektion des betreffenden Antigens oder gegebenenfalls auch schon an einer hieraus hervorgegangenen Infektionserkrankung leidet. Werden demgegenüber keine ASCs nachgewiesen, stammen die getesteten eukaryotischen Zellen in aller Regel von menschlichen oder tierischen Organismen, die entweder an einer latenten Infektion, insbesondere an einer bereits über Monate persistierenden Infektion, des betreffenden Antigens leiden oder aber unter medizinischen Gesichtpunkten als gesund gelten. Unter einem gesunden menschlichen oder tierischen Organismus soll ein Organismus verstanden werden, der entweder eine Infektion überstanden hat oder aber zu keinem Zeitpunkt an einer Infektion litt.

Gewöhnlich stammen die eukaryotischen Zellen aus einer menschlichen und/oder tierischen Probe. Bevorzugt stammen die eukaryotische Zellen aus Proben von Körperflüssigkeiten, insbesondere Blutproben, Cervix-Abstrichen und/oder Bronchiallavagen.

In einer bevorzugten Ausführungsform werden die eukaryotischen Zellen, insbesondere unter den eukaryotischen Zellen vorkommende immunkompetente Zellen, sogenannte Immunzellen, vor der Inkubation angereichert. Bei den eukaryotischen Zellen handelt es sich bevorzugt um Blutzellen, insbesondere um B-Lymphozyten, T-Lymphozyten, Granulozyten, dendritische Zellen, Makrophagen und/oder Erythrozyten. Zur Anreicherung der eukaryotischen Zellen kommen die üblicherweise verwendeten zellulären Anreicherungstechniken in Betracht. So können die eukaryotischen Zellen beispielsweise durch Zentrifugation, insbesondere Gradienten-Zentrifugation, angereichert werden. Bei der Gradienten-Zentrifugation können beispielsweise Zucker-Gradienten eingesetzt werden. Bevorzugt werden die eukaryotischen Zellen vor der Inkubation von Erythrozyten befreit. In einer möglichen Ausführungsform werden unter den eukaryotischen Zellen vorkommende ASCs angereichert.

In einer besonders bevorzugten Ausführungsform werden die eukaryotischen Zellen vor der Inkubation von Blutserum, insbesondere autologem Blutserum, befreit. Dies geschieht gewöhnlich ausgehend von einer geronnenen Blutprobe durch Abtrennung des flüssigen Blutanteils von zellulären Blutbestandteilen. Bezüglich der zellulären Bestandteile und geeigneter Abtrennungstechniken wird auf den vorherigen Absatz Bezug genommen. Mit der Abtrennung des Blutserums werden mit besonderem Vorteil störende Serumbestandteile, insbesondere Serumproteine, wie beispielsweise Albumine und/oder Immunoglobuline, insbesondere Antikörper, entfernt. Diese können ansonsten zu verfälschten Testergebnissen führen.

In einer weitergehenden Ausführungsform werden Fragmente von Erregertypen, insbesondere Erregerepitope, als Antigen verwendet. Bei den Epitopen handelt es sich insbesondere um Peptide, vorzugsweise Oligopeptide. Geeignete Epitope können aus 5 bis 25, insbesondere 9 bis 11, Aminosäureeinheiten aufgebaut sein.

Erfindungsgemäß ist es insbsondere vorgesehen, dass zur Inkubation der eukaryotischen Zellen zumindest ein Antigen aus der Gruppe PPD, RD 1, RD 2, ESAT 6, CFP 10, MPT 41, MTB 64, PPE 44, OSP A, OSP B, OSP C, OSP D, OSP E, VIsE, p 58, p 100 und Dbp A, verwendet wird.

Zur Inkubation der eukaryotischen Zellen wird zumindest ein Tuberkulose spezifisches Antigen, insbesondere aus der Gruppe PPD, RD 1, RD 2, MPT 64, MTB 41 und PPE 44, eingesetzt.

Alternativ oder in Kombination wird zur Inkubation der eukaryotischen Zellen zumindest ein Borreliose spezifisches Antigen, insbesondere aus der Gruppe VlsE, OSP A, OSP B, OSP C, OSP D und OSP E, verwendet.

Das erfindungsgemäße Verfahren wird gewöhnlich auf einer hierfür geeigneten Untersuchungsfläche, insbesondere in einem Untersuchungsgefäß, bzw. auf einem hierfür geeigneten Träger durchgeführt. Bei der Untersuchungsfläche bzw. dem Träger kann es sich beispielsweise um Loch- oder Vertiefungsböden von sogenannten Lochplatten, WellPlatten oder Mikrotiterplatten handeln. Derartige Loch- bzw. Mikrotiterplatten, die bevorzugt als Untersuchungsgefäße verwendet werden, sind kommerziell erhältlich, insbesondere mit unterschiedlichen Loch- bzw. Vertiefungszahlen (Wells). Beispielsweise kann das erfindungsgemäße Verfahren mit Hilfe einer 96-Well Mikrotiterplatte durchgeführt werden. Die Mikrotiterplatten können beispielsweise Vertiefungsdurchmesser von ca. 5 mm aufweisen, was einer Grundfläche von etwa 20 mm² entspricht. Die verwendete Untersuchungsfläche ist zweckmäßigerweise eben. Dadurch können lokale Konzentrationsunterschiede bei der Durchführung des erfindungsgemäßen Verfahrens vermieden werden. Außerdem eignen sich ebene Untersuchungsflächen grundsätzlich besser für die Erzeugung von zellulären Einfachschichten (Monolayer). Typische Materialien, aus denen geeignete Untersuchungsgefäße bestehen können, sind beispielsweise Polystyrol, Polyvinylidendifluorid (PVDF), Nitrocellulose oder Nylon.

In einer besonders geeigneten Ausführungsform wird die Anzahl der Antigen spezifische Antikörper sezernierenden Zellen gemessen. Bezüglich der hierzu in Frage kommenden Messmethoden wird auf die folgende Beschreibung verwiesen.

Wie bereits erwähnt, ist es erfindungsgemäß besonders bevorzugt, wenn die Anzahl der Antigen spezifische Antikörper sezernierenden Zellen gemessen wird.

In einer besonders bevorzugten Ausführungsform wird das Verfahren als ELISPOT-Verfahren (Enzyme Linked Immuno Spot Technique) durchgeführt. Mit anderen Worten, erfindungsgemäß ist es besonders bevorzugt, wenn es sich bei dem Verfahren um ein ELISPOT-Verfahren handelt. Das ELISPOT-Verfahren wird im Gegensatz zum ELISA-Verfahren an fester Phase, in der Regel auf einem hierfür geeigneten Träger, durchgeführt. Bei dem Träger kann es sich um die bereits erwähnten Lochplatten oder Mikrotiterplatten bzw. deren Loch- oder Vertiefungsböden handeln. Mit Hilfe der ELISPOT-Methode kann allgemein die Sezernierung (Absonderung bzw. Ausscheidung) von Zytokinen und/oder Antikörpern aus Zellen unter Verwendung von Antigenen oder Antikörpern gemessen werden, die diese Zytokine bzw. Antikörper spezifisch binden. Die abgesonderten Zytokine bzw. Antikörper werden gewöhnlich auf Vertiefungsböden von Mikrotiterplatten in Form von farbigen Punkten, sogenannten Spots, sichtbar gemacht. Durch Auszählung der Spots und/oder durch Bestimmung ihrer Farbintensitäten mittels geeigneter Software-Programme können Aussagen über die Aktivität der Zellen gemacht werden.

Das im Rahmen der Erfindung verwendbare ELISPOT-Verfahren umfasst in einer weitergehenden Ausführungsform die folgenden Schritte:
a) Aufbringen von Abfangmolekülen auf eine Untersuchungsfläche bzw. auf einen Träger,
b) Inkubieren der eukaryotischen Zellen mit dem Antigen auf der Untersuchungsfläche bzw. auf dem Träger,
c) Zugabe von Detektionsmolekülen auf die Untersuchungsfläche bzw. auf den Träger,
d) Detektion der ASCs auf der Untersuchungsfläche bzw. auf dem Träger, deren sezernierte Antikörper spezifisch gegen das Antigen gerichtet sind.

Die aufgebrachten Abfangmoleküle können in der Regel an die Untersuchungsfläche binden und werden dadurch mit besonderem Vorteil auf dieser fixiert. Die Abfangmoleküle können insbesondere schichtförmig auf die Untersuchungsfläche aufgebracht werden. Als Abfangmoleküle werden bevorzugt Antikörper (Abfang-Antikörper) verwendet, die gegen Antikörper oder Antikörper-Subtypen der nachzuweisenden ASCs gerichtet sind. Beispielsweise kann es sich bei den Antikörper-Subtypen im Falle von PPD als Antigen um zumindest einen der folgenden Suptypen aus der Gruppe IgG1, IgG2, IgG3 und IgG4 handeln. Man spricht in diesem Zusammenhang auch von sogenannten Anti-Antikörpern. Bei den Abfangmolekülen kann es sich um polyklonale oder monklonale Antikörper handeln, wobei monoklonale Antikörper bevorzugt sind. Bevorzugt wird zum Aufbringen der Abfangmoleküle auf die Untersuchungsfläche eine wässrige Dispersion der Abfangmoleküle verwendet. Zur Herstellung der wässrigen Dispersion können die Abfangmoleküle in einem geeigneten Puffer, beispielsweise einem Acetatpuffer, dispergiert werden. Gegebenenfalls kann die Dispersion zum Erhalt einer Lösung filtriert werden. In der Regel wird die wässrige Dispersion erst unmittelbar vor dem Aufbringen der Abfangmoleküle auf die Untersuchungsfläche hergestellt. Bevorzugt weisen die Abfangmoleküle in der wässrigen Dispersion eine Konzentration zwischen 1 und 3 µg/ml³, insbesondere ca. 2,5 µg/ml³, auf.

Die eukaryotischen Zellen werden normalerweise in Form einer Zellsuspension auf die Untersuchungsfläche aufgebracht. Das Antigen wird gewöhnlich als wässrige Lösung auf die Untersuchungsfläche aufgebracht. Erfindungsgemäß ist es insbesondere vorgesehen, dass das Antigen vor den eukaryotischen Zellen auf die Untersuchungsfläche aufgebracht wird. Üblicherweise wird das Antigen auf der Untersuchungsfläche fixiert, insbesondere direkt am Boden der Untersuchungsfläche gekoppelt. In diesem Fall muss kein zusätzliches Antigen zur Inkubation der eukaryotischen Zellen hinzugegeben werden.
Die Inkubation der eukaryotischen Zellen wird während eines Zeitraumes zwischen 2 und 18 Stunden, insbesondere 2 und 4 Stunden, durchgeführt. Dadurch wird mit besonderem Vorteil erreicht, dass sich noch keine Gedächtniszellen gegen das Antigen ausbilden, die ansonsten zu einer Verfälschung der Testergebnisse führen würden. Somit wird gewährleistet, dass nur die Zellen erfasst werden, die für eine akute bzw. aktive Infektion verantwortlich sind. Durch die Inkubation mit dem Antigen werden unter den eukaryotischen Zellen vorhandene ASCs, deren Antikörper spezifisch gegen das Antigen gerichtet sind, zur Sezernierung entsprechender Antikörper veranlasst. Diese werden durch die Abfangmoleküle abgefangen und so auf der Untersuchungsfläche gebunden.

Des Weiteren können erfindungsgemäß Waschschritte vorgesehen sein. Die Untersuchungsfläche kann bspw. vor der Zugabe der Detektionsmoleküle gewaschen werden. Zum Waschen der Untersuchungsfläche werden üblicherweise Pufferlösungen, beispielsweise Phosphatpuffer, verwendet. Dadurch werden ungebundene Bestandteile, insbesondere eukaryotische Zellen, von der Untersuchungsfläche entfernt. Gegebenenfalls kann das Waschen der Untersuchungsfläche wiederholt werden, unter Umständen auch mehrfach.

Als Detektionsmoleküle wird normalerweise ein weiterer Antikörpertyp, ein sogenannter Detektions-Antikörper, verwendet. Die Detektionsmoleküle sind ebenfalls spezifisch gegen Antikörper der nachzuweisenden ASCs gerichtet. Typischerweiseweise binden die Detektionsmoleküle jedoch an anderen Stellen der ASC-Antikörper als die zuvor beschriebenen Abfangmoleküle. Dadurch entstehen ternäre Komplexe aus Abfangmolekülen, ASC-Antikörpern und Detektionsmolekülen auf der Untersuchungsfläche. Bevorzugt werden die Detektionsmoleküle als Konjugatverbindungen eingesetzt. Als mögliche Konjugationspartner kommen Enzyme, Fluoreszenzfarbstoffe, Gold und/oder Silber in Frage. Enzyme, insbesondere alkalische Phosphatase oder Meerettich-Peroxidase, insbesondere equinen Ursprungs, und/oder GlucoseOxidase sind bevorzugt. Geeignete Beispiele für Fluoreszenzfarbstoffe sind Fluoreszeinisothiocyanat und/oder Cyanine 3. Daneben kommen aber auch eine Reihe weiterer Farbstoffe in Betracht. Derartige Farbstoffe sind dem Fachmann hinreichend bekannt. Ein weiterer bevorzugter Konjugationspartner ist Biotin. Ein biotinyliertes Detektionsmolekül wird üblicherweise zusammen mit einer Konjugatverbindung aus Avidin oder Streptavidin und einem geeigneten Konjugationspartner, beispielsweise alkalische Phosphatase oder Meerettich-Peroxidase, verwendet.

Weiterhin kann die Untersuchungsfläche vor der Detektion der ASCs gewaschen werden. Dadurch können nicht gebundene Detektionsmoleküle von der Untersuchungsfläche entfernt werden. Zum Waschen der Untersuchungsfläche können Waschlösungen, insbesondere Pufferlösungen, verwendet werden. Gegebenenfalls kann der Waschschritt wiederholt, insbesondere mehrfach wiederholt, werden.

Gewöhnlich wird die Detektion der ASCs durch eine kolorimetrische Nachweisreaktion vorgenommen. Durch die Nachweisreaktion entstehen farbige Spots (Färbungen) auf der Untersuchungsfläche, die gegebenenfalls miteinander überlappen können. In Ausnahmefällen kann auch die gesamte Untersuchungsfläche gefärbt sein. Die Spots können entweder mit einem Mikroskop oder einem automatischen Bildanalyse-System detektiert und insbesondere ausgezählt werden. Der Vergleich der Anzahl von Spots mit der eingesetzten Zellzahl erlaubt es, die Frequenz oder Anzahl reagierender Zellen zu errechnen. Bevorzugt wird die Nachweisreaktion durch ein Enzym katalysiert. Die Nachweisreaktion kann beispielsweise mit Hilfe geeigneter Substrate, insbesondere von Chromogenen, durchgeführt werden. Als geeignete Substrate werden beispielhaft Para-Nitrophenylphosphat, Carbazol oder Brom-Chlor-Indolyl-Phosphat genannt. Beispielsweise spaltet alkalische Phosphatase den Phosphatrest vom farblosen p-Nitrophenylphosphat ab, wodurch das gelb gefärbte p-Nitrophenolat entsteht. Die Reaktion kann beispielsweise mit einem Photometer verfolgt werden. Die Intensität der Farbe ist dabei proportional zur Konzentration des p-Nitrophenolations und damit auch zur Konzentration der Antikörper der nachzuweisenden ASCs.

In einer bevorzugten Ausführungsform des erfindungsgemäßen Verfahrens wird die Anzahl der Antigen spezifische Antikörper sezernierenden Zellen (ASCs) gemessen. Grundsätzlich kann hierzu die Anzahl von auf der Untersuchungsfläche auftretenden Spots bestimmt werden. Alternativ oder in Kombination dazu wird bevorzugt die Farbintensität einzelner Spots oder überlappender Spots, gegebenenfalls auch von Färbungen, die die gesamte Untersuchungsfläche bedecken, quantitativ gemessen. Besonders bevorzugt wird die Untersuchungsfläche in eine Vielzahl von Einzelpunkten zerlegt, die Farbintensität jedes Einzelpunktes getrennt gemessen und die gemessenen Intensitätswerte addiert. Erfindungsgemäß kann es insbesondere vorgesehen sein, dass 1 bis 2 Millionen, insbesondere ca. 1,5 Millionen, Bildpunkte (Pixel) pro Untersuchungsfläche erfasst werden. Die Erfassung der Bildpunkte kann beispielsweise mit Hilfe einer Kamera erfolgen. Für die Messung der Farbintensität eines Einzelpunktes auf der Untersuchungsfläche stehen mit besonderem Vorteil zwischen 200 und 300, insbesondere zwischen 220 und 260, vorzugsweise ca. 256, Abstufungen (Grauwerte) zur Verfügung. Die Verarbeitung und Auswertung der Bildpunkte kann beispielsweise mit einem Lesegerät vorgenommen werden. Vorzugsweise wird die Gesamtzahl der Bildpunkte sowie insbesondere ihre Intensität mit Hilfe eines sogenannten Image-Analyzers gemessen. Dadurch ist eine Messung der gesamten Färbung, bezogen auf die Untersuchungsfläche oder einen bestimmten Teil davon, möglich. Als Referenzwert kann eine ungefärbte Stelle auf der Untersuchungsfläche, gegebenenfalls auch einer anderen Untersuchungsfläche, dienen. Das Maß für die Gesamtaktivität, d.h. die Gesamtintensität der Reaktionen aller eukaryotischen Zellen auf der Untersuchungsfläche auf ein definiertes Antigen, ergibt sich dann aus der Anzahl der angeregten (gefärbten) Pixel, multipliziert mit dem Farbwert (beispielsweise Graustufe zwischen 0 und 256) jedes angeregten Pixels. Dieses Produkt wird zweckmäßigerweise durch 1000 geteilt, um einfach handhabbare Zahlenwerte (Einheiten) zu erhalten. Die Erfassung und Auswertung der Bildpunkte erfolgt vorzugsweise von oben, d.h. oberhalb der Untersuchungsfläche. Die Verarbeitung zu einem zweidimensionalen Bild erfolgt gewöhnlich mit Hilfe von Computertechniken.

Gewöhnlich werden für die Bilderzeugung geeignete Filtersysteme eingesetzt. Bevorzugt kommen Filtersysteme, insbesondere Filtersätze, mit Schmalbandfiltern zum Einsatz. Erfindungsgemäß kann es insbesondere vorgesehen sein, dass zum Testen der eukrayotischen Zellen auf ASCs mit einem Schmalband-Anregungsfilter und einem Schmalband-Sperrfilter pro verwendetem Filtersatz gearbeitet wird. Die Filtersätze können zusammen mit einem Strahlenleiter zu einem Filterblock integriert sein.

In einer weiteren Ausführungsform können Maßnahmen ergriffen werden, wodurch eine auf der Untersuchungsfläche erzeugte Färbung in der Intensität pro Einzelpunkt im Vergleich zum Stand der Technik verringert und/oder in der Flächenausdehnung, d.h. der Zahl der Einzelpunkte pro Zelle, vergrößert wird. Durch die Vergrößerung der Flächenausdehnung wird insbesondere die Färbung pro Flächeneinheit schwächer und somit Färbungsunterschiede zwischen den einzelnen Flächen und/oder innerhalb einer größeren Fläche deutlicher. Auf diese Weise können die Farbintensitäten der einzelnen Punkte in den technisch messbaren Bereich geführt werden. Damit können auch intensiv gefärbte Flächen, deren Intensität den technisch erfassbaren Maximalwert überschritten haben, aufgrund der Vergrößerung der Flächenausdehnung erfasst werden. Durch die in den vorhergehenden Abschnitten beschriebene Messmethode kann die Einschätzung des Infektionsstatus eines Patienten zusätzlich verbessert werden.

Das erfindungsgemäße Verfahren eignet sich vor allem zur Überprüfung des Infektionsstatus zur Unterscheidung von akuten Infektionen bzw. Infektionserkrankungen einerseits und latenten oder
überstandenen Infektionen bzw. Infektionserkrankungen andererseits. Die Bezeichnungen zur Charakterisierung des Immunstatus können abhängig von den Infektionen bzw. Infektionserkrankungen variieren. So wird im Zusammenhang mit der Tuberkulose von akuten und latenten Infektionen gesprochen. Dagegen werden bei der Borreliose gewöhnlich die Bezeichnungen aktive und chronische Borreliose verwendet. Weiterhin eignet sich das erfindungsgemäße Verfahren auch zur Überprüfung des Impfschutzes, d.h. es kann mit Hilfe des erfindungsgemäßen Verfahrens beurteilt werden, ob ein noch ausreichender Impfschutz vorliegt oder eine Nachimpfung erforderlich ist.

Bei den Infektionen und/oder Infektionserkrankungen handelt es sich um Tuberkulose und/oder Borreliose

Die vorliegende Erfindung betrifft weiterhin die Verwendung eines Kits (wie in Anspruch 12 beschrieben) zur in vitro-Diagnose und/oder in vitro-Therapieverfolgung von Infektionen und/oder Infektionserkrankungen zur Unterscheidung zwischen akuten Infektionen einerseits und latenten oder überstandenen Infektionen andererseits, umfassend zumindest eine Komponente zum Nachweis von Antigen spezifischen Antikörper sezernierenden Zellen (ASCs). Als Kitkomponenten kommen insbesondere Abfangmoleküle, Detek-tionsmoleküle Chemolumineszenzfarbstoffe, Fluoreszenzfarbstoffe und/oder Antigene in Frage. Die Komponenten können in Form von wässrigen Dispersionen und/oder in getrockneter Form, beispielsweise lyophilisierter Form, vorliegen. Umfasst das Kit zwei oder mehr Komponenten, liegen diese bevorzugt räumlich getrennt voneinander vor. Bezüglich weiterer Merkmale und Eigenschaften des Kits wird auf die bisherige Beschreibung Bezug genommen.

Weitere Einzelheiten und Merkmale der Erfindung ergeben sich aus der nachfolgenden Beschreibung bevorzugter Ausführungsformen in Form von Beispielen in Verbindung mit den Unteransprüchen. Hierbei können die jeweiligen Merkmale für sich alleine oder zu mehreren in Kombination miteinander verwirklicht sein.

### Beispiel 1:

### Nachweis von Tuberkulose-Erreger

Zur Durchführung des Verfahrens wird eine 96-Lochplatte verwendet. Zunächst werden Anti-Antikörper als Abfangmoleküle auf den Böden der Vertiefungen der Lochplatte immobilisiert. Die verwendeten Anti-Antikörper sind dabei spezifisch gegen die Antikörper von Tuberkulose-Erreger spezifische Antikörper sezernierenden Zellen gerichtet. Danach wird PPD als Tuberkulose-Erreger auf dem Boden der Vertiefungen immobilisiert. Zusätzlich oder alternativ dazu können auch Peptide vom sogenannten RD1- und RD2-Komplex benutzt werden. Des Weiteren können auch weitere Proteine von Mycobacterium Tuberculosum (38 kD, 41 kD, 44 kD, 64 kD) eingesetzt werden. Anschließend werden pro Vertiefung 100.000 bis 250.000 PBMCs (peripheral blood mononuclear cells) hinzugegeben. Die Inkubation der Blutzellen (zusammen mit den Tuberkulose-Erregern) wird während eines Zeitraums von ca. 18 Stunden bei ca. 37 °C vorgenommen. Danach werden die Zellen dekantiert und die Lochplatte mehrfach gewaschen. Anschließend wird ein weiterer Anti-Antikörper hinzugegeben, der mit alkalischer Phosphatase, konjugiert ist (detection capture molecule). Es wird eine erneute Inkubation bei ca. 37 °C während 4 bis 12 Stunden vorgenommen. Anschließend werden nicht gebundene konjugierte Anti-Antikörper von dem Lochplattenboden der 96-Lochplatte abgespült. Nach Zugabe von Para-Nitrophenylphosphat werden die Untersuchungsflächen (Böden der Vertiefungen) auf auftretende Färbungen hin untersucht. Treten keine farbigen Spots (Verfärbungen) auf, ist die Reaktion negativ. Dies bedeutet, dass keine Tuberkulose spezifischen Plasmaplasten (ASCs) unter den Blutzellen vorhanden sind. Damit hat der Patient, dessen Blutzellen untersucht wurden, keine akute oder aktive Tuberkulose. Er ist entweder diesbezüglich negativ oder hat eine latente oder überstandene, nicht aktive Tuberkulose.

### Beispiel 2:

### Nachweis von Borrelien

Zur Durchführung des Verfahrens wird eine 96-Lochplatte verwendet. Auf den Böden der Vertiefungen der Lochplatte werden Anti-Antikörper immobilisiert, die spezifisch gegen die Antikörper von Borrelien spezifische Antikörper sezernierenden Zellen gerichtet sind. Anschließend werden Borrelien spezifische Antigene, beispielsweise die Peptide OSP A, OSP B, OSP C und/oder VIsE inneres Flaggelin Fragment benutzt. Die Borrelien spezifischen Antigene werden mit einer Konzentration von 1 bis 10 µg/ml auf den Böden der Vertiefungen gekoppelt. Danach werden pro Vertiefungen 100.000 bis 250.000 PBMCs (peripheral blood mononuclear cells) hinzugegeben. Die Blutzellen werden (zusammen mit den Borrelien spezifischen Antigenen) während eines Zeitraums von ca. 18 Stunden bei ca. 37 °C inkubiert. Danach werden die Blutzellen dekantiert und die Lochplatte mehrfach gewaschen. Anschließend wird ein weiterer Anti-Antikörper hinzugegeben, der mit alkalischer Phosphatase konjugiert ist. Es wird erneut eine Inkubation bei ca. 37 °C während 4 bis 12 Stunden vorgenommen. Anschließend werden nicht gebundene konjugierte Antikörper von der Platte gewaschen. Nach Zugabe von Para-Nitrophenylphosphat werden die Böden der Vertiefungen auf auftretende Verfärbungen untersucht. Treten farbige Spots auf den Böden der Vertiefungen auf, so sind damit Borrelien spezifische ASCs unter den untersuchten Blutzellen nachgewiesen. In diesem Fall lautet die Diagnose auf akute bzw. aktive Borreliose. Treten dagegen keine Verfärbungen auf, bedeutet dies entweder eine latente oder überstandene Borreliose.

## Patentansprüche

1. In vitro-Verfahren zur Diagnose und/oder Therapieverfolgung von Tuberkulose und/oder Borreliose zur Unterscheidung zwischen akuten (aktiven) Infektionen einerseits und latenten (chronischen) oder überstandenen Infektionen andererseits, **dadurch gekennzeichnet, dass** eukaryotische Zellen mit zumindest einem Tuberkulose spezifischen Antigen und/oder zumindest einem Borreliose spezifischen Antigen inkubiert werden und auf Antigen spezifische Antikörper sezernierende Zellen (ASCs) getestet werden, deren sezernierte Antikörper spezifisch gegen das Antigen gerichtet sind, wobei die Inkubation der eukaryotischen Zellen während eines Zeitraumes zwischen 2 und 18 Stunden durchgeführt wird.

2. Verfahren nach Anspruch 1, **dadurch gekennzeichnet, dass** die eukaryotischen Zellen vor der Inkubation angereichert werden.

3. Verfahren nach Anspruch 1 oder 2, **dadurch gekennzeichnet, dass** die eukaryotischen Zellen vor der Inkubation von Erythrozyten befreit werden.

4. Verfahren nach einem der vorhergehenden Ansprüche, **dadurch gekennzeichnet, dass** die eukaryotischen Zellen vor der Inkubation von Blutserum, insbesondere autologem Blutserum, befreit werden.

5. Verfahren nach einem der vorhergehenden Ansprüche, **dadurch gekennzeichnet, dass** zur Inkubation der eukaryotischen Zellen Erregerepitope als Antigen verwendet werden.

6. Verfahren nach einem der vorhergehenden Ansprüche, **dadurch kennzeichnet, dass** zur Inkubation der eukaryotischen Zellen zumindest ein Antigen aus der Gruppe PPD, RD 1, RD 2, ESAT 6, CFP 10, MPT 41, MTB 64, PPE 44, OSP A, OSP B, OSP C, OSP D, OSP E und VIsE eingesetzt wird.

7. Verfahren nach einem der vorhergehenden Ansprüche, **dadurch gekennzeichnet, dass** zur Inkubation der eukaryotischen Zellen zumindest ein Tuberkulose spezifisches Antigen aus der Gruppe PPD, RD 1, RD 2, MPT 64, MTB 41 und PPE 44 verwendet wird.

8. Verfahren nach einem der vorhergehenden Ansprüche, **dadurch gekennzeichnet, dass** zur Inkubation der eukaryotischen Zellen zumindest ein Borreliose spezifisches Antigen aus der Gruppe VIsE, OSP A, OSP B, OSP C, OSP D und OSP E eingesetzt wird.

9. Verfahren nach einem der vorhergehenden Ansprüche, **dadurch gekennzeichnet, dass** das Verfahren als ELISPOT-Verfahren durchgeführt wird, insbesondere umfassend die Schritte:
a) Aufbringen von Abfangmolekülen auf eine Untersuchungsfläche,
b) Inkubieren der eukaryotischen Zellen mit dem Antigen auf der Untersuchungsfläche,
c) Zugabe von Detektionsmolekülen auf die Untersuchungsfläche,
d) Detektion von ASCs auf der Untersuchungsfläche, deren sezernierte Antikörper spezifisch gegen das Antigen gerichtet sind.

10. Verwendung des Verfahrens nach einem der vorhergehenden Ansprüche zur Überprüfung des Impfschutzes.

11. Verwendung des Verfahrens nach einem der Ansprüche 1 bis 9 zur Beurteilung des Immunstatus, insbesondere zur Unterscheidung zwischen akuten Infektionen einerseits und latenten oder überstandenen Infektionen andererseits.

12. Verwendung eines Kits, umfassend zumindest eine Komponente zum Nachweis von Antigen spezifischen Antikörper sezernierenden Zellen (ASCs), insbesondere Abfangmoleküle, Detektionsmoleküle, Chemolumineszenzfarbstoffe, Fluoreszenzfarbstoffe und/oder Antigene, in einem Verfahren nach einem der Ansprüche 1 bis 9.

## Claims

1. Method for the in-vitro diagnosis and/or in-vitro therapy monitoring of tuberculosis and/or borreliosis for differentiation between acute (active) infections on the one hand and latent (chronic) or overcome infections on the other hand, **characterized in that** eukaryotic cells are incubated with at least one tuberculosis specific antigen and/or at least one borreliosis specific antigen and tested for cells (ASCs) secreting antigen-specific antibodies, the secreted antibodies of which are directed specifically against the antigen, wherein incubation of the eukaryotic cells is for a time period between 2 and 18 hours.

2. Method according to claim 1, **characterized in that** the eukaryotic cells are enriched before incubation.

3. Method according to claim 1 or 2, **characterized in that** the eukaryotic cells are freed from erythrocytes before incubation.

4. Method according to any of the preceding claims, **characterized in that** the eukaryotic cells are freed from blood serum, in particular autologous blood serum, before incubation.

5. Method according to any of the preceding claims, **characterized in that** for incubation of the eukaryotic cells epitopes of a pathogenic type are used as the antigen.

6. Method according to any of the preceding claims, **characterized in that** for incubation of the eukaryotic cells at least one antigen from the group PPD, RD 1, RD 2, ESAT 6, CFP 10, MPT 41, MTB 64, PPE 44, OSP A, OSP B, OSP C, OSP D, OSP E and VIsE is employed.

7. Method according to any of the preceding claims, **characterized in that** for incubation of the eukaryotic cells at least one tuberculosis specific antigen from the group PPD, RD 1, RD 2, MPT 64, MTB 41 and PPE 44 is used.

8. Method according to any of the preceding claims, **characterized in that** for incubation of the eukaryotic cells at least one borreliosis specific antigen from the group VlsE, OSP A, OSP B, OSP C, OSP D and OSP E is employed.

9. Method according to any of the preceding claims, **characterized in that** the method is carried out as the ELISPOT method, in particular comprising the steps:
a) application of capture molecules to an investigation surface,
b) incubation of the eukaryotic cells with the antigen on the investigation surface,
c) addition of detection molecules to the investigation surface,
d) detection of the ASCs on the investigation surface, the secreted antibodies of which are directed specifically against the antigen.

10. Use of the method according to any of the preceding claims for examining the immunization protection.

11. Use of the method according to any of claims 1 to 9 for assessment of the immune status, in particular for differentiation between acute infections on the one hand and latent or overcome infections on the other hand.

12. Use of a kit, comprising at least one component for the detection of antigen-specific antibody-secreting cells (ASCs), in particular capture molecules, detection molecules, chemoluminescent dyes, fluorescent dyes and/or antigens, in a method according to any of claims 1 to 9.

## Revendications

1. Procédé in vitro destiné au diagnostic et/ou au suivi du traitement de la tuberculose et/ou de la borréliose en vue de faire la distinction entre des infections aiguës (actives), d'une part, et des infections latentes (chroniques) ou qui ont été surmontées, d'autre part, **caractérisé en ce que** des cellules eucaryotes, avec tout au moins un antigène spécifique de la tuberculose et/ou tout au moins un antigène spécifique de la borréliose, sont incubées et testées pour des cellules sécrétant des anticorps (ASC) spécifiques d'un antigène, cellules dont les anticorps sécrétés sont dirigés spécifiquement contre l'antigène, l'incubation des cellules eucaryotes étant réalisée sur une période comprise entre 2 et 18 heures.

2. Procédé selon la revendication 1, **caractérisé en ce que** les cellules eucaryotes sont enrichies avant l'incubation.

3. Procédé selon la revendication 1 ou 2, **caractérisé en ce que** les cellules eucaryotes sont libérées des érythrocytes avant l'incubation.

4. Procédé selon l'une des revendications précédentes, **caractérisé en ce que** les cellules eucaryotes sont libérées du sérum sanguin avant l'incubation, en particulier du sérum sanguin autologue.

5. Procédé selon l'une des revendications précédentes, **caractérisé en ce que** des épitopes pathogènes sont employés comme antigène en vue de l'incubation des cellules eucaryotes.

6. Procédé selon l'une des revendications précédentes, **caractérisé en ce que** tout au moins un antigène, issu du groupe PPD, RD 1, RD 2, ESAT 6, CFP 10, MPT 41, MTB 64, PPE 44, OSP A, OSP B, OSP C, OSP D, OSP E et VlsE, est utilisé en vue de l'incubation des cellules eucaryotes.

7. Procédé selon l'une des revendications précédentes, **caractérisé en ce que** tout au moins un antigène spécifique de la tuberculose, issu du groupe PPD, RD 1, RD 2, MPT 64, MTB 41 et PPE 44, est employé en vue de l'incubation des cellules eucaryotes.

8. Procédé selon l'une des revendications précédentes, **caractérisé en ce que** tout au moins un antigène spécifique de la borréliose, issu du groupe VlsE, OSP A, OSP B, OSP C, OSP D et OSP E, est utilisé en vue de l'incubation des cellules eucaryotes.

9. Procédé selon l'une des revendications précédentes, **caractérisé en ce que** le procédé est réalisé sous la forme d'un procédé ELISPOT, lequel comprend en particulier les phases suivantes :
a) l'introduction de molécules de capture sur une surface d'analyse;
b) l'incubation des cellules eucaryotes avec l'antigène sur la surface d'analyse;
c) l'adjonction de molécules de détection sur la surface d'analyse;
d) la détection des ASC sur la surface d'analyse, dont les anticorps sécrétés sont spécifiquement dirigés contre l'antigène.

10. Utilisation du procédé selon l'une des revendications précédentes en vue de vérifier la protection vaccinale.

11. Utilisation du procédé selon l'une des revendications 1 à 9 en vue de l'appréciation du statut immunitaire, en particulier en vue de faire la distinction entre des infections aiguës, d'une part, et des infections latentes ou qui ont été surmontées, d'autre part.

12. Utilisation d'un kit, comprenant tout au moins un composant destiné à prouver la présence de cellules sécrétant des anticorps (ASC) spécifiques d'un antigène, en particulier des molécules de capture, des molécules de détection, des colorants à luminescence chimique, des colorants fluorescents et/ou des antigènes, dans un procédé selon l'une des revendications 1 à 9.
